# EUROPÄISCHE PATENTSCHRIFT

(11) **EP 1 221 436 B1**
(45) Veröffentlichungstag und Bekanntmachung des Hinweises auf die Patenterteilung: **18.04.2007**
(21) Anmeldenummer: 01130515.8
(22) Anmeldetag: 21.12.2001
(51) Int. Cl.: C07C 209/48, C07C 211/11

(54) **Verfahren zur Herstellung von sekundären Aminen aus Nitrilen**
Process for the preparation of secondary amines from nitriles
Procédé pour la préparation d'amines secondaires à partir de nitriles

(30) Priorität: 05.01.2001 DE 10100314
(43) Veröffentlichungstag der Anmeldung: 10.07.2002
(73) Patentinhaber: BASF Aktiengesellschaft, 67056 Ludwigshafen (DE)
(72) Erfinder: Pfeffinger, Joachim, 67063 Ludwigshafen (DE); Hüllmann, Michael, Dr., 64625 Bensheim (DE); Höhn, Arthur, Dr., 67281 Kirchheim (DE); Funke, Frank, Dr., 68159 Mannheim (DE); Ohlbach, Frank, Dr., 40219 Düsseldorf (DE); Gerlach, Till, Dr., 67071 Ludwigshafen (DE)
(74) Vertreter: Isenbruck, Günter

(56) Entgegenhaltungen:
- DE-A- 3 935 641
- US-A- 3 117 162
- S. MORRIS KUPCHAN ET AL.: "Tumor Inhibitors. 70. Structure-Cytotoxicity Relationships among N-Acyltriamines Related to Solapalmitine" JOURNAL OF MEDICINAL CHEMISTRY., Bd. 15, Nr. 1, Januar 1972 (1972-01), Seiten 65-68, XP002214904 AMERICAN CHEMICAL SOCIETY. WASHINGTON., US ISSN: 0022-2623

## Beschreibung

Die Erfindung betrifft ein Verfahren zur Herstellung von sekundären Aminen aus Nitrilen an einem Rhodium-Katalysator.

Verfahren zur Herstellung von Aminen aus Nitrilen an bestimmten RhodiumKatalysatoren sind bekannt.

US 3,117,162 betrifft die Hydrierung von Nitrilen, wobei Kupplungsreaktionen minimiert werden sollen. Als Katalysatoren können rhodiumhaltige Katalysatoren eingesetzt werden, wobei bei Drücken im Bereich von 1 bis 50 Atmosphären gearbeitet wird. Beispielsweise werden 5% Rhodium auf Kohle als Katalysator eingesetzt. Neben primären Aminen werden auch sekundäre Amine erhalten, siehe Tabelle V. Die Umsetzung wird dabei in einem inerten Lösungsmittel durchgeführt.

US 5,574,189 betrifft die Hydrierung von Nitrilen zur Herstellung von Aminen. Dabei sollen insbesondere sekundäre Amine hergestellt werden. Während überwiegend multimetallische Katalysatoren beschrieben werden, ist zum Vergleich auch ein Katalysator aus 1% Rh auf Al₂O₃ beschrieben, siehe Tabelle V. Die Umsetzung wird dabei bei 500 psi (34 bar) Druck durchgeführt. Es werden sowohl primäre, sekundäre als auch tertiäre aliphatische Amine erhalten.

J. Med. Chem. 15, 65 - 68 (1972) beschreibt unter anderem die Hydrierung von tertiären Nitrilen an einem Rh-Al₂O₃-Katalysator. Insbesondere erfolgt die Herstellung von Bis-DMAPA durch Hydrierung von DMAPN in Gegenwart von 5 % Rh-Al₂O₃ als Katalysator.

Aufgabe der vorliegenden Erfindung ist die Bereitstellung eines Verfahrens zur Herstellung von sekundären Aminen in hohen Ausbeuten und Selektivitäten durch Umsetzung von Nitrilen mit Wasserstoff in Gegenwart von Katalysatoren.

Insbesondere sollen symmetrisch aufgebaute sekundäre Amine hergestellt werden.

Die Aufgabe wird erfindungsgemäß gelöst durch ein Verfahren zur Herstellung von sekundären Aminen der allgemeinen Formel (II)

(X-CH₂-)₂NH (II)

mit der Bedeutung
- X: ein gegebenenfalls durch C₁₋₂₀-Alkyl, C₃₋₈-Cycloalkyl, C₄₋₂₀-Alkylcycloalkyl, C₄₋₂₀-Cycloalkylalkyl, C₂₋₂₀-Alkoxyalkyl, C₆₋₁₄-Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy und/oder C₂₋₈-Alkoxycarbonyl substituiertes C₁₋₂₀-Alkyl, C₂₋₂₀-Alkenyl oder C₃₋₈-Cycloalkyl
durch Umsetzung von Nitrilen der allgemeinen Formel (III)

X-CN (III)

mit Wasserstoff bei Temperaturen von 20 bis 250°C und Drücken von 60 bis 350 bar in Gegenwart eines Rh enthaltenden Katalysators, wobei der Katalysator, bezogen auf das Gesamtgewicht des Katalysators, 0,1 bis 5 Gew.% Rh auf einem Träger enthält, zu Gemischen aus primären Aminen der allgemeinen Formel (I)

X-CH₂-NH₂ (I)

und sekundären Aminen der allgemeinen Formel (II)

(X-CH₂-)₂NH (II)

und zumindest teilweise Rückführung der von den erhaltenen Gemischen abgetrennten primären Amine in die Umsetzung.

Dabei wird die Menge der in die Umsetzung zurückgeführten primären Amine so gewählt, daß sie der Menge der nach der Umsetzung vorliegenden primären Amine im wesentlichen entspricht. Dies ist durch Einsatz des erfindungsgemäßen Katalysators möglich.

Das erfindungsgemäße Verfahren erlaubt die Herstellung von sekundären Aminen in hohen Ausbeuten, insbesondere ohne Anfall an auszuschleusenden primären Aminen.

Erfindungsgemäß wurde gefunden, daß die Verwendung eines wie vorstehend definierten Katalysators zur Umsetzung von Nitrilen mit Wasserstoff zu sekundären Aminen zudem zu einer längeren Standzeit bzw. Langzeitstabilität des Katalysators führen kann.

Die erfindungsgemäß eingesetzten Katalysatoren enthalten, bezogen auf das Gesamtgewicht des Katalysators, 0,1 bis 5 Gew.-%, vorzugsweise 0,3 bis 3 Gew.-%, besonders bevorzugt 0,5 bis 1 Gew.-% Rhodium.

Zudem können sie, bezogen auf das Gesamtgewicht des Katalysators, 0,1 bis 10 Gew.-%, vorzugsweise 0,1 bis 5 Gew.-%, besonders bevorzugt 0,2 bis 1 Gew.-% mindestens eines weiteren Metalls enthalten, ausgewählt aus den Gruppen IB und VIII des Periodensystems der Elemente, Cer und Lanthan. Es kann ein weiteres Metall oder ein Gemisch mehrerer weiterer Metalle eingesetzt werden. Bevorzugt werden in diesem Fall Kupfer, Platin, Palladium und Gemische daraus, besonders bevorzugt Platin verwendet.

Vorzugsweise weist der Katalysator Rh als alleinige Aktivkomponente auf. Besonders bevorzugt besteht der Katalysator aus Rh auf dem Träger.

Als Träger können alle bekannten geeigneten Träger verwendet werden. Beispielsweise ist der Träger ausgewählt aus Aktivkohle, Siliciumcarbid und Metalloxiden. Dabei werden als Metalloxide vorzugsweise Zeolithe, Aluminiumoxid, Siliciumdioxid, Titandioxid, Zirkoniumdioxid, Zinkoxid, Magnesiumoxid oder deren Gemische verwendet, die gegebenenfalls mit Alkali- und/oder Erdalkalimetalloxiden dotiert sind. Besonders bevorzugt werden γ-Aluminiumoxid, Siliciumdioxid, Zirkoniumdioxid oder Titanoxid oder Gemische davon eingesetzt, insbesondere γ-Al₂O₃ und ZrO₂. Die Träger können in beliebiger Form eingesetzt werden, beispielsweise als Extrudate (in Form von Strängen), Pellets oder Tabletten.

Besonders bevorzugt ist ein Katalysator, der etwa 0,5 bis 1,0 Gew.-% Rh, bezogen auf das Gesamtgewicht des Katalysators, auf γ-Al₂O₃ oder ZrO₂ als Träger enthält.

Die Katalysatoren können nach allgemein bekannten Verfahren hergestellt werden, beispielsweise durch Tränken des Trägers mit Lösungen von Verbindungen der eingesetzten Metalle.

Die Träger können beispielsweise mit Metall-Vorläufern beschichtet werden. Als Metallvorläufer eignen sich Metallsalze, wie Nitrate, Nitrosylnitrate, Halogenide, Carbonate, Carboxylate, Acetylacetonate, Chlorokomplexe, Nitritokomplexe und Aminkomplexe des Rhodiums. Bevorzugt sind Nitrate, Chloride, Chlorokomplexe und Aminkomplexe, insbesondere Nitrate. Das Aufbringen erfolgt vorzugsweise durch Tränken. Die Metallvorläufer der Metalle (sofern mehrere Metalle vorliegen) können gleichzeitig oder nacheinander aufgetränkt werden. Die Reihenfolge, in der die Aktivkomponenten aufgetränkt werden, ist beliebig wählbar.

Bevorzugt wird die Tränkung des Trägermaterials mit einer Lösung eines Rhodiumsalzes in einem geeigneten Lösungsmittel, bevorzugt in Wasser. Beim Tränken des Trägermaterials mit der rhodiumhaltigen Lösung kann die Menge der verwendeten Lösung so bemessen werden, daß sie etwa 80 bis 100% der Fähigkeit zur Wasseraufnahme des Trägermaterials entspricht. Die Konzentration an Rhodium in der Lösung wird dann so gewählt, daß die gewünschte Beladung des Trägermaterials mit Rhodium erzielt wird. Das getränkte Trägermaterial wird bei Temperaturen zwischen 50 und 200°C, bevorzugt zwischen 100 und 140°C, getrocknet. Das Trocknen kann bewegt oder ruhend und über eine Zeitdauer von 0,5 bis 10 h, bevorzugt 4 bis 6 h, geschehen. Das Calcinieren des getrockneten Katalysators erfolgt bei Temperaturen zwischen 300 und 800°C, bevorzugt zwischen 400 und 600°C und kann ebenfalls bewegt oder ruhend durchgeführt werden. Der calcinierte Katalysator ist vor dem Einsatz in der Synthese zu reduzieren, dies kann durch eine Wasserstoffbehandlung bei Temperaturen zwischen 100 und 300°C, bevorzugt zwischen 180 und 220°C, bei Normaldruck und über eine Zeitdauer von 2 bis 20 h, bevorzugt 8 bis 14 h, nach Einbau des Katalysators in den Synthesereaktor durchgeführt werden.

Weitere Verfahren zur Herstellung der erfindungsgemäß verwendeten Katalysatoren sind dem Fachmann bekannt und schließen das Bedampfen, Besputtern und gemeinsame Fällen ein.

Die Oberfläche, das Porenvolumen und die Porengrößenverteilung des Katalysators sind in weiten Bereichen unkritisch.

Das erfindungsgemäße Verfahren wird bei Temperaturen von 20 bis 250°C, vorzugsweise 50 bis 200°C, besonders bevorzugt 100 bis 140°C und Drücken von 60 bis 350 bar, vorzugsweise 70 bis 200 bar, besonders bevorzugt 70 bis 120 bar diskontinuierlich oder bevorzugt kontinuierlich in Druckapparaturen wie Autoklaven oder bevorzugt in einem Rohrreaktor durchgerührt. Der Druck ist dabei vorzugsweise der Wasserstoffdruck im Reaktor. Bei der Verwendung eines Rohrreaktors kann der verwendete Katalysator auch als Festbettkatalysator vorliegen.

Die Umsetzung wird vorzugsweise in der Flüssigphase in Sumpf- oder Rieselfahrweise, besonders bevorzugt in Sumpffahrweise durchgeführt. Dabei wird insbesondere bevorzugt ohne Verwendung von Ammoniak gearbeitet.

Die Katalysatorbelastung, bezogen auf das eingesetzte Nitril, beträgt vorzugsweise 0,1 bis 2 kg/(l h), insbesondere etwa 0,6 kg/(l h). Dabei kann (neben dem primären Amin) zusätzlich ein Teil des flüssigen Reaktionsaustrages in die Umsetzung zurückgeführt werden.

Das erfindungsgemäße Verfahren läßt sich lösungsmittelfrei oder in Lösungsmitteln, wie Wasser, Methanol, Ethanol, Tetrahydrofuran, Methyl-tert.-butylether oder N-Methylpyrrolidon durchführen. Im Lösungsmittel kann dabei das Nitril der allgemeinen Formel (III) gelöst sein. Vorzugsweise wird lösungsmittelfrei gearbeitet.

Die im erfindungsgemäßen Verfahren erhaltenen Amine der allgemeinen Formeln (I) und (II) lassen sich in an sich bekannter Weise, beispielsweise destillativ, vom Reaktionsgemisch abtrennen und reinigen.

Beispielsweise ist es möglich, durch Rektifikation einen Strom mit reinem sekundärem Amin und einen Strom mit primärem Amin zu erhalten, wobei der Strom mit dem primären Amin zumindest teilweise in die Synthese zurückgeführt wird. Dabei wird soviel primäres Amin zurückgeführt, daß es weder zu einer Aufpegelung noch zu einer Abpegelung kommt.

Im erfindungsgemäßen Verfahren werden Nitrile der allgemeinen Formel (III) umgesetzt.

X-CN (III)

- X: ist dabei ein gegebenenfalls durch C₁₋₂₀-Alkyl, C₃₋₈-Cycloalkyl, C₄₋₂₀-Alkylcycloalkyl, C₄₋₂₀-Cycloalkylalkyl, C₂₋₂₀-Alkoxyalkyl, C₆₋₁₄Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂₀-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy und/oder C₂₋₈-Alkoxycarbonyl substituiertes C₁₋₂₀-Alkyl, C₂₋₂₀-Alkenyl oder C₃₋₈-Cycloalkyl.

X ist dabei vorzugsweise C₁₋₁₂-, besonders bevorzugt C₁₋₈-, insbesondere C₁₋₆-, speziell C₁₋₄-Alkyl, das verzweigt oder unverzweigt sein kann und vorzugsweise unverzweigt ist. Beispiele sind unverzweigte Reste aus 1, 2, 3, 4, 5, 6, 7, 8, 9, 10, 11, 12, 13, 14, 15, 16, 17, 18, 19, 20 Methyleneinheiten, C(C)-C-C, C-C(C)-C, C-C(C)₂-C als Struktureinheiten. Bevorzugt sind als Struktureinheiten C, C-C, C-C-C, C-C-C-C, C-C-C-C-C-C, C-C(C)-C-C, C-C(C)-C-C, C-C-C(CN)-C-C-C, besonders bevorzugt C, C-C, C-C-C, C-C-C-C.

X kann, wie vorstehend angegeben, substituiert sein. Dabei kann die Anzahl der Substituenten bis zur Anzahl der substituierbaren Wasserstoffatome in X betragen. Abhängig von der Art des Restes können 1 bis 5, vorzugsweise 1 bis 3, insbesondere 0, 1 oder 2 Substituenten vorliegen. Als Substituenten kommen in Betracht:
- C₁₋₂₀-Alkoxy, bevorzugt C₁₋₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁₋₄-Alkoxy wie Methoxy, Ethoxy, n-Propoxy, iso-Propoxy, n-Butoxy, iso-Butoxy, sec.-Butoxy und tert.-Butoxy,
- Hydroxy,
- C₁₋₂₀-Hydroxyalkyl, bevorzugt C₁₋₈-Hydroxyalkyl, besonders bevorzugt C₁₋₄-Hydroxyalkyl wie Hydroxymethyl, 1-Hydroxyethyl, 2-Hydroxyethyl, 2-Hydroxy-2-propyl und 3-Hydroxy-n-propyl,
- Amino,
- C₁₋₂₀-Alkylamino, bevorzugt C₁₋₈-Alkylamino besonders bevorzugt C₁₋₄-Alkylamino wie Methylamino, oder entsprechende Aminoalkyl, 1-Aminoethyl, 2-Aminoethyl, 2-Amino-n-propyl und 3-Amino-n-propyl,
- C₂₋₂₀-Dialkylamino, bevorzugt C₂₋₁₂-Dialkylamino, besonders C₂₋₈-Dialkylamino wie N,N-Dimethylamino, N,N-Diethylamino, N,N-Dipropylamino, N,N-Di-(1-methylethyl)amino, N,N-Dibutylamino, N,N-Di-(1-methylpropyl)-amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)-amino, N-Butyl-N-methylamino, N-Methyl-N-(1-methylpropyl)amino, N-Methyl-N-(2-methylpropyl)amino, N-(1,1-Dime-thylethyl)-N-methylamino, N-Ethyl-N-propylamino, N-Ethyl-N-(1-methyl-ethyl)-amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methyl-propyl)-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, n-(1-Methylethyl)-N-(1-methylpropyl)-amino, N-(1Methylethyl)-N-(1-methpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methyl-propyl)amino, n-(1,1-Dimethylethyl)-N-(1-methylpropyl)amino und N-(1,1-Diemthylethyl)-N-(2-methylpropyl)amino,
- C₃₋₁₂-Azacycloalkyl, bevorzugt C₃₋₈-Azacycloalkylamino, besonders bevorzugt C₅₋₈-Azacycloalkyl wie Pyrrolidin, Piperidin, Azepan, Piperazin, N-Alkylpiperazin und Morpholin,
- C₃₋₈-Cycloalkylamino wie Cyclopropylamino, Cyclobutylamino, Cyclopentylamino, Cyclohexylamino, Cycloheptylamino und Cyclooctylamino, bevorzugt Cyclopentylamino, Cyclohexylamino und Cyclooctylamino, besonders bevorzugt Cyclopentylamino und Cyclohexylamino,
- C₃₋₈-Dicycloalkylamino,
- Arylamino wie Phenylamino, 1-Naphthylamino und 2-Naphthylamino, bevorzugt Phenylamino,
- Aryl-C₁₋₈-alkylamino, bevorzugt Phenyl-C₁₋₈-alkylamino, besonders bevorzugt Phenyl-C₁₋₄-alkylamino wie Phenylmethylamino und Phenylethylamino,
- Halogen, bevorzugt Fluor, Chlor und Brom, besonders bevorzugt Fluor und Chlor,
- Mercapto,
- C₂₋₂₀-Oxacycloalkyl, bevorzugt C₂₋₈-Oxacycloalkyl, besonders bevorzugt C₂₋₈-Oxacycloalkyl, wie 2-Tetrahydrofuranyl, 3-Tetrahydrofuranyl, 2-Furanyl und 3-Furanyl,
- C₃₋₈-Cycloalkoxy wie Cyclopropoxy, Cyclobutoxy, Cyclopentoxy, Cyclohexoxy, Cycloheptoxy und Cyclooctoxy, bevorzugt Cyclopentoxy, Cyclohexoxy, besonders bevorzugt Cyclopentoxy und Cyclohexoxy,
- Aryloxy wie Phenoxy, 1-Naphthoxy und 2-Naphthoxy, bevorzugt Phenoxy.

Vorzugsweise liegen 0, 1 oder 2 Substituenten vor, die OH oder C₂₋₁₂-, vorzugsweise C₂₋₆-, insbesondere C₂₋₄-Dialkylamino sind. Insbesondere sind die Substituenten Dimethylamino oder OH.

Bevorzugte Nitrile der allgemeinen Formel (III) sind Acetonitril, Propionitril, Isopropionitril, Butyronitril, Valeronitril, Pentensäurenitril, Retensäurenitril, 3-Hydroxypropionitril, 3-Methoxypropionitril, 3-Ethoxypropionitril, 3-Propoxypropionitril, 3-Isopropoxypropionitril, 3-Cyclohexoxypropionitril, 2-Methyl-3-hydroxypropionitril, 3-Methoxy-2-methylpropionitril, 3-Ethoxy-2-methylpropionitril, 2-Methyl-3-propoxypropionitril, 3-Isopropoxy-2-methylpropionitril, 3-Cyclohexoxy-2-methylpropionitril, 3-Methyl-3-Hydroxypropionitril, 3-Methoxy-3-methylpropionitril, 3-Ethoxy-3-methylpropionitril, 3-Methyl-3-propoxypropionitril, 3-Isopropoxy-3-methylpropionitril, 3-Cyclohexoxy-3-methyl-propionitril, 3-Aminopropionitril, 3-Methylaminopropionitril, 3-Dimethylaminopropionitril, 3-Ethylaminopropionitril, 3-Diethylaminopropionitril, 3-Propylaminopropionitril, 3-Dipropylaminopropionitril, 3-Isopropylamino-propionitril, 3-Diisopropylaminopropionitril, 3-Cyclohexylaminopropionitril, 3-Dicyclohexylaminopropionitril, N-(Cyanoethyl)-N-methylanilin. Besonders bevorzugt sind 3-Hydroxypropionitril, 3-Methoxypropionitril, 3-Dimethyl-aminopropionitril, 3-Diethylaminopropionitril, 3-Cyclohexylaminopropionitril und 3-Methylaminopropionitril, insbesondere Biscyanethylether, Biscyanethylamin, N-Methylbiscyanethylamin, N-Ethyl-biscyanethylamin, N-n-Propyl-biscya-nethylamin, N-n-Propylbiscyanethylamin, Polyisobutylennitril, N-Polyiso-butylenaminopropionitril, Triscyanethylamin, 5-Aminovaleriansäurenitril, 5-Methylaminovaleriansäurenitril, 5- Dimethylaminovaleriansäurenitril, 6-Amino-capronsäurenitril, 6-Methylaminocapronsäurenitril, 6-Dimethylamino-capronsäurenitril, 5-Amino-4-methylvaleriansäurenitril, 5-Methyl-amino-4-methylvaleriansäurenitril, 5-Dimethylamino-4-methylvaleriansäurenitril, 5-Ethylamino-4-methylvaleriansäurenitril, 5-Diethylamino-4-methylvaleriansäurenitril, 5-Amino-2-methylvaleriansäurenitril, 5-Methylamino-2-methylvaleriansäurenitril, 5-Dimethylamino-2-valeriansäurenitril, 5-Ethylamino-2-methylvaleriansäurenitril, 5-Diethylamino-2-methylvaleriansäurenitril, 4-Cyanokorksäuredinitril, Acrylnitril.

Insbesondere bevorzugt sind Adipodinitril, 3-Dimethylaminopropionitril (DMAPN) und 3-Hydroxypropionitril, speziell DMAPN.

Die sekundären Amine, vorzugsweise Bis-DMAPA, sind Härter für Epoxidharze, Katalysatoren für Polyurethane, Zwischenprodukte zur Herstellung quartärer Ammoniumverbindungen, Weichmacher, Korrosionsinhibitoren, Textilhilfsmittel, Farbstoffe und Emulgatoren. Mehrfach funktionalisierte tertiäre Amine dienen außerdem zur Herstellung von Kunstharzen, Ionenaustauschern, Pharmazeutika, Pflanzenschutz- und Schädlingsbekämpfungsmitteln.

Die Erfindung wird durch die nachstehenden Beispiele näher erläutert.

### Beispiel 1 (Referenz)

Ein elektrisch beheizter Rohrreaktor mit 30 mm Innendurchmesser, einem zentral angebrachten Thermoelement mit 12 mm Durchmesser und einer Gesamtlänge von 2,0 m wird mit einer Mischung aus 286 g (=500 ml) des Katalysators und 450 ml Edelstahlfüllkörpern gefüllt. Der Katalysator besteht aus 1,0 Gew.-% Rh auf γ-Al₂O₃ als Trägermaterial und hat die Form von Strängen mit 2 mm Durchmesser und 5 bis 10 mm Länge.

Vor der Reaktion wird der Katalysator drucklos über einen Zeitraum von 12 Stunden mit reinem Wasserstoff bei 180°C reduziert.

Durch den Reaktor werden im Gleichstrom von unten nach oben 400 g/h an 3-(Dimethylamino)-propionitril (DMAPN), 1,5 kg/h an flüssigem Reaktionsaustrag und 0,4 Nm³/h Wasserstoff zugegeben. Die Edukte werden vor dem Reaktor auf eine Temperatur von 120°C erhitzt. Der Reaktor wird bei einer Temperatur von 120°C und einem Gesamtdruck von 200 bar gehalten.

Das aus dem Reaktor austretende Gemisch wird abgekühlt, ein Teil der Flüssigkeit wird an den Reaktoreingang zurückgeführt, und der übrige Teil wird auf Normaldruck entspannt. Mittels gaschromatographischer Analyse wurde im Reaktoraustrag ein Gehalt von 63 Gew.-% an Bis-(3-dimethylaminopropyl)-amin (Bis-DMAPA), 29 Gew.-% an 3-Dimethylaminopropylamin (DMAPA), 0,3 Gew.% an DMAPN sowie 7,7 Gew.% an verschiedenen Nebenprodukten gefunden. Der Umsatz an DMAPN lag damit bei 99,7%, die Selektivität von gebildeten Bis-DMAPA bezogen auf eingesetztes DMAPN lag bei 62%.

### Beispiel 2 (Referenz)

Es wurde verfahren wie in Beispiel 1 beschrieben, jedoch wurden 595 g (= 500 ml) eines Katalysators mit 0,5 Gew.-% Rh auf ZrO₂ als Trägermaterial eingesetzt. Der Katalysator hatte die Form von Ringen mit 7 mm Durchmesser, 3 mm Lochdurchmesser und 3 mm Dicke.

Durch den Reaktor werden im Gleichstrom von unten nach oben 300 g/h an 3-(Dimethylamino)-propionitril (DMAPN, 2,5 kg/h an flüssigem Reaktionsaustrag und 0,4 Nm³/h Wasserstoff zugegeben. Der Reaktor wurde bei einer Temperatur von 130°C und einem Gesamtdruck von 200 bar betrieben. Es wurde ein Reaktionsaustrag mit einem Gehalt von 69 Gew.-% an Bis-(3-dimethylaminopropyl)-amin (Bis-DMAPA), 26 Gew.-% an 3-Dimethylaminopropylamin (DMAPA), 0,9% an DMAPN sowie 4,1 Gew.-% an verschiedenen Nebenprodukten hatte. Der Umsatz an DMAPN lag damit bei 99,1%.

### Beispiel 3

Durch den Reaktor aus Beispiel 1 werden im Gleichstrom von unten nach oben 300 g/h an 3-(Dimethylamino)-propionitril (DMAPN), 125 g/h an zurückgewonnenem Dimethylaminopropylamin (DMAPA), 1,5 kg/h an flüssigem Reaktionsaustrag und 0,4 Nm³/h Wasserstoff zugegeben. Der Reaktor wurde bei einer Temperatur von 140°C und einem Gesamtdruck von 100 bar betrieben. Es wurde ein Reaktionsaustrag mit einem Gehalt von 54 Gew.-% an Bis-(3-dimethylaminopropyl)-amin (Bis-DMAPA), 35 Gew.-% an 3-Di-methylaminopropylamin (DMAPA), 0,1% an DMAPN erhalten. Das Produktgemisch wurde diskontinuierlich aufdestilliert, wobei zunächst bei Normaldruck DMAPA mit einer Reinheit von 99,5% und einer Destillationsausbeute von 90% gewonnen wurde, welches in die Synthese zurückgeführt wurde. Anschließend wurde Bis-DMAPA bei einem Gesamtdruck von 50 mbar mit einer Reinheit von 99,2% und einer Destillationsausbeute von 88% gewonnen. Die molare Gesamtausbeute an Bis-DMAPA bezogen auf eingesetztes DMAPN liegt damit einschließlich der Destillation bei ca. 67%.

### Beispiel 4

Ein elektrisch beheizter Rohrreaktor mit 20 mm Innendurchmesser, einem zentral angebrachten Thermoelement mit 3 mm Durchmesser und einer Gesamtlänge von 0,31 m wird mit einer Mischung aus 61 g (= 100 ml) Katalysator gefüllt. Der Katalysator besteht aus 0,5 Gew.-% Rh auf γ-Al₂O₃ als Trägermaterial und hat die Form von dünnen Strängen mit sternförmigem Querschnitt und 5-10 mm Länge.

Vor der Reaktion wird der Katalysator drucklos über einen Zeitraum von 12 Stunden mit reinem Wasserstoff bei 300°C reduziert.

Durch den Reaktor werden im Gleichstrom von unten nach oben 3-(Dimethylamino)propionitril (DMAPN) und Dimethylaminopropylamin (DMAPA) mit den in Tabelle 1 angegebenen Volumenströmen, 39 g/h an flüssigem Reaktionsaustrag sowie 20 1/h Wasserstoff gegeben. Der Reaktor wird bei einer Temperatur von 120°C und einem Gesamtdruck von 80 bar gehalten.

Das aus dem Reaktor austretende Gemisch wird abgekühlt und auf Normaldruck entspannt. Die in Tabelle 1 angegebenen Produktströme an DMAPN, DMAPA und Bis-DMAPA werden aus der gaschromatographischen Analyse des Austrages errechnet.

Die Daten in Tabelle 1 zeigen, daß bei den angegebenen Bedingungen Zulauf-Verhältnisse von DMAPN zu DMAPA gefunden werden können, bei denen die DMAPA-Bilanz nahezu null wird (letzte Spalte in Tabelle 1). Die Bis-DMAPA-Selektivität bezogen auf DMAPN liegt dann bei über 90%.

**Tabelle 1**

| Zulauf | | | | Austrag | | | Austrag-Eintrag |
|---|---|---|---|---|---|---|---|
| DMAPN | DMAPA | DMAPN | DMAPA | DMAPN | DMAPA | Bis-DMAPA | DMAPA |
| [g/h] | [g/h] | [%] | [%] | [g/h] | [g/h] | [g/h] | [g/h] |
| 19,0 | 20,3 | 48 | 52 | 0,23 | 18,9 | 18,8 | -1,4 |
| 27,3 | 12,5 | 69 | 31 | 0,44 | 12,6 | 24,0 | 0,1 |
| 37,9 | 4,7 | 89 | 11 | 0,14 | 10,6 | 27,9 | 5,9 |

## Patentansprüche

1. Verfahren zur Herstellung von sekundären Aminen der allgemeinen Formel (II)
(X-CH₂-)₂NH (II)
mit der Bedeutung
X ein gegebenenfalls durch C₁₋₂₀-Alkyl, C₃₋₈-Cycloalkyl, C₄₋₂₀-Alkylcycloalkyl, C₄₋₂₀-Cycloalkylalkyl, C₂₋₂₀-Alkoxyalkyl, C₆₋₁₄-Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂₀-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy und/oder C₂₋₈-Alkoxycarbonyl substituiertes C₁₋₂₀-Alkyl, C₂₋₂₀-Alkenyl oder C₃₋₈-Cycloalkyl
durch Umsetzung von Nitrilen der allgemeinen Formel (III)
X-CN (III)
mit Wasserstoff bei Temperaturen von 20 bis 250°C und Drücken von 60 bis 350 bar in Gegenwart eines Rh enthaltenden Katalysators, wobei der Katalysator, bezogen auf das Gesamtgewicht des Katalysators, 0,1 bis 5 Gew.% Rh auf einem Träger enthält, zu Gemischen aus primären Aminen der allgemeinen Formel (I)
X-CH₂-NH₂ (I)
und sekundären Aminen der allgemeinen Formel (II)
(X-CH₂-)₂NH (II)
und zumindest teilweise Rückführung der von den erhaltenen Gemischen abgetrennten primären Amine in die Umsetzung,
wobei die Menge der in die Umsetzung zurückgeführten primären Amine so gewählt wird, daß sie der Menge der nach der Umsetzung vorliegenden primären Amine im wesentlichen entspricht.

2. Verfahren nach Anspruch 1, **dadurch gekennzeichnet, daß** der Katalysator 0,3 bis 3 Gew.% Rh als alleinige Aktivkomponente enthält.

3. Verfahren nach einem der Ansprüche 1 oder 2, **dadurch gekennzeichnet, daß** der Träger ausgewählt ist aus Aktivkohle, Siliciumcarbid und Metalloxiden.

4. Verfahren nach Anspruch 3, **dadurch gekennzeichnet, daß** der Träger γ-Al₂O₃ oder ZrO₂ ist.

5. Verfahren nach einem der Ansprüche 1 bis 4, **dadurch gekennzeichnet, daß** X lineares C₁₋₆-Alkyl mit bis zu 2 Substituenten ist.

6. Verfahren nach Anspruch 5, **dadurch gekennzeichnet, daß** die Substituenten C₂₋₁₂-Dialkylamino oder OH sind.

7. Verfahren nach Anspruch 6, **dadurch gekennzeichnet, daß** das Nitril der allgemeinen Formel (III) 3-(Dimethylamino)piopionitril (DMAPN) ist, das zu einem Gemisch aus Bis-(3-(dimethylamino)propyl)amin (Bis-DMAPA) und 3-(Dimethylamino)propylamin (DMAPA) umgesetzt wird, wobei Bis-DMAPA als Produkt gewonnen wird und das gebildete DMAPA zumindest teilweise in die Umsetzung zurückgeführt wird.

## Claims

1. A process for preparing secondary amines of the formula (II)
(X-CH₂-)₂NH (II)
where
X is a C₁₋₂₀-alkyl, C₂₋₂₀-alkenyl or C₃₋₈-cycloalkyl group which may optionally be substituted by C₁₋₂₀-alkyl, C₃₋₈-cycloalkyl, C₄₋₂₀-alkylcycloalkyl, C₄₋₂₀-cycloalkylalkyl, C₂₋₂₀-alkoxyalkyl, C₆₋₁₄-aryl, C₇₋₂₀-alkylaryl, C₇₋₂₀-aralkyl, C₁₋₂₀-alkoxy, hydroxy, C₁₋₂₀-hydroxyalkyl, amino, C₁₋₂₀-alkylamino, C₂₋₂₀-dialkylamino, C₂₋₁₂-alkenylamino, C₃₋₈-cycloalkylamino, arylamino, diarylamino, aryl-C₁₋₈-alkylamino, halogen, mercapto, C₂₋₂₀-alkenyloxy, C₃₋₈-cycloalkoxy, aryloxy and/or C₂₋₈-alkoxycarbonyl,
by reacting nitriles of the formula (III)
X-CN (III)
with hydrogen at from 20 to 250°C and pressures of from 60 to 350 bar in the presence of an Rh- comprising catalyst comprising from 0.1 to 5% by weight, based on the total weight of the catalyst, of Rh on a support to give mixtures of primary amines of the formula (I)
X-CH₂-NH₂ (I)
and secondary amines of the formula (II)
(X-CH₂-)₂NH (II)
and returning at least part of the primary amines separated from the mixtures obtained to the reaction, with the amount of primary amines returned to the reaction being selected so that it corresponds essentially to the amount of primary amines present after the reaction.

2. The process according to claim 1, wherein the catalyst comprises from 0.3 to 3% by weight of Rh as the only active component.

3. The process according to either claims 1 or 2, wherein the support is selected from among activated carbon, silicon carbide and metal oxides.

4. The process according to claim 3, wherein the support is γ-Al₂O₃ or ZrO₂.

5. The process according to any of claims 1 to 4, wherein X is linear C₁₋₆-alkyl having up to 2 substituents.

6. The process according to claim 5, wherein the substituents are C₂₋₁₂-dialkylamino or OH.

7. The process according to claim 6, wherein the nitrile of the formula (III) is 3-(dimethylamino)propionitrile (DMAPN) and is converted into a mixture of bis(3-(dimethylamino)propyl)amine (bis-DMAPA) and 3-(dimethylamino)propylamine (DMAPA), with bis-DMAPA being isolated as product and at least part of the DMAPA formed being returned to the reaction.

## Revendications

1. Procédé de préparation d'amines secondaires de formule générale (II)
(X-CH₂-)₂NH (II)
avec les significations suivantes :
X est un alkyle en C₁₋₂₀, un alcényle en C₂₋₂₀ ou un cycloalkyle en C₃₋₈ éventuellement substitué par les suivants: alkyle en C₁₋₂₀, cycloalkyle en C₃₋₈, alkylcycloalkyle en C₄-₂₀, cycloalkylalkyle en C₄₋₂₀, alcoxyalkyle en C₂₋₂₀, aryle en C₆₋₁₄, alkylaryle en C₇₋₂₀, aralkyle en C₇₋₂₀, alcoxy en C₁₋₂₀, hydroxy, hydroxyalkyle en C₁₋₂₀, amino, alkylamino en C₁₋₂₀, dialkylamino en C₂₋₂₀, alcénylamino en C₂₋₁₂, cycloalkylamino en C₃₋₈, arylamino, diarylamino, aryl-alkylamino en C₁₋₈, halogène, mercapto, alcényloxy en C₂₋₂₀, cycloalcoxy en C₃₋₈, aryloxy et/ou alcoxycarbonyle en C₂₋₈,
par réaction de nitriles de formule générale (III)
X-CN (III)
avec de l'hydrogène à des températures de 20 à 250°C et à des pressions de 60 à 350 bars en présence d'un catalyseur contenant du Rh, le catalyseur, par rapport au poids total du catalyseur, contenant de 0,1 à 5% en poids de Rh sur un support, pour obtenir des mélanges d'amines primaires de formule générale (I)
X-CH₂-NH₂ (I)
et d'amines secondaires de formule générale (II)
(X-CH₂-)₂NH (II)
et recyclage au moins partiel des amines primaires séparées par les mélanges obtenus dans la réaction, la quantité des amines primaires recyclées dans la réaction étant choisie de telle sorte qu'elle corresponde essentiellement à la quantité d'amines primaires présentes après la réaction.

2. Procédé selon la revendication 1, **caractérisé en ce que** le catalyseur contient de 0,3 à 3% en poids de Rh comme composant actif unique.

3. Procédé selon l'une des revendications 1 ou 2, **caractérisé en ce que** le support est choisi parmi le charbon actif, le carbure de silicium et les oxydes de métaux.

4. Procédé selon la revendication 3, **caractérisé en ce que** le support est du γ-Al₂O₃ ou du ZrO₂.

5. Procédé selon l'une des revendications 1 à 4, **caractérisé en ce que** X est un alkyle en C₁₋₆ linéaire avec 2 substituants maximum.

6. Procédé selon la revendication 5, **caractérisé en ce que** les substituants sont un dialkylamino en C₂₋₁₂ ou OH.

7. Procédé selon la revendication 6, **caractérisé en ce que** le nitrile de formule générale (III) est du 3-(diméthylamino)propionitrile (DMAPN), lequel est mis à réagir pour obtenir un mélange composé de bis-(3-(diméthylamino)propyl)amine (bis-DMAPA) et de 3-(diméthylamino)propylamine (DMAPA), le bis-DMAPA étant obtenu comme produit et le DMAPA formé est recyclé au moins partiellement dans la réaction.
